# EUROPÄISCHE PATENTSCHRIFT

(11) **EP 3 122 318 B1**
(45) Veröffentlichungstag und Bekanntmachung des Hinweises auf die Patenterteilung: **14.03.2018**
(21) Anmeldenummer: 15702697.2
(22) Anmeldetag: 27.01.2015
(51) Int. Cl.: A61K 8/35, A61Q 5/02, A61Q 19/00, A61K 8/81, A61Q 19/08, A61Q 19/10

(54) **VERDICKTE TRANSPARENTE TENSIDSYSTEME MIT FLIESSGRENZE, ENTHALTEND 4-HYDROXYACETOPHENON**
THICKENED TRANSPARENT SURFACTANT SYSTEMS HAVING A FLOW LIMIT, CONTAINING 4-HYDROXYACETOPHENONE
SYSTÈMES DE TENSIOACTIFS TRANSPARENTS ÉPAISSIS, PRÉSENTANT UN SEUIL D'ÉCOULEMENT, CONTENANT DU 4-HYDROXYACÉTOPHÉNONE

(30) Priorität: 26.03.2014 DE 102014104261
(43) Veröffentlichungstag der Anmeldung: 01.02.2017
(73) Patentinhaber: Symrise AG, 37603 Holzminden (DE)
(72) Erfinder: PRUNS, Julia, 20144 Hamburg (DE); VETTER, Katrin, 23843 Bad Oldesloe (DE); RASCHKE, Thomas, 25421 Pinneberg (DE); VON WEDEL-PARLOW, Magdalena, 20251 Hamburg (DE); ARGEMBEAUX, Horst, 21465 Wentorf (DE); WEBER, Susanne, 22335 Hamburg (DE)
(74) Vertreter: Fabry, Bernd
(86) Internationale Anmeldenummer: PCT/EP2015/051550
(87) Internationale Veröffentlichungsnummer: WO 2015/144330

(56) Entgegenhaltungen:
- EP-A1- 2 774 481
- EP-A1- 2 774 604
- WO-A1-01/19946
- WO-A1-2005/030163
- JP-A- H07 206 645
- KR-A- 20130 134 976
- SYMRISE: "Multiple Benefits for Cosmetics with SymSave H", INTERNET CITATION, 26. Juli 2013 (2013-07-26), XP007923019, Gefunden im Internet: URL:http://www.symrise.com/newsroom/articl e/multiple-benefits-for-cosmetics-with-sym saveR-h/ [gefunden am 2015-02-25]
- RAJABI L ET AL: "Acetophenones with selective antimycobacterial activity", LETTERS IN APPLIED MICROBIOLOGY, OXFORD, GB , Bd. 40, Nr. 3 1. März 2005 (2005-03-01), Seiten 212-217, XP002693043, ISSN: 1472-765X, DOI: 10.1111/J.1472-765X.2005.01657.X Gefunden im Internet: URL:http://onlinelibrary.wiley.com/doi/10. 1111/j.1472-765X.2005.01657.x/full [gefunden am 2013-02-18]

## Beschreibung

Die vorliegende Erfindung betrifft transparente Tensidsysteme, insbesondere kosmetische Reinigungsmittel. Derartige Mittel sind an sich bekannt. Es handelt sich dabei im Wesentlichen um oberflächenaktive Substanzen oder Stoffgemische, die dem Verbraucher in verschiedenen Zubereitungen angeboten werden.

Zubereitungen dieser Art sind beispielsweise Schaum- und Duschbäder, feste und flüssige Seifen oder sogenannte "Syndets" (synthetische Detergentien), Shampoos, Handwaschpasten, Intimwaschmittel, spezielle Reinigungsmittel für Kleinkinder und dergleichen.

Oberflächenaktive Stoffe - am bekanntesten die Alkalisalze der höheren Fettsäuren, also die klassischen "Seifen" - sind amphiphile Stoffe, die organische unpolare Substanzen in Wasser emulgieren können.

Diese Stoffe schwemmen nicht nur Schmutz von Haut und Haaren, sie reizen, je nach Wahl des Tensids oder des Tensidgemisches, Haut und Schleimhäute mehr oder minder stark.

Das gebräuchlichste Tensid für kosmetische Zusammensetzungen ist das Natriumlaurylethersulfat. Obwohl es gute Waschkraft besitzt und gut haut- und schleimhautverträglich ist, sollten empfindliche Personen den häufigen Kontakt damit meiden.

Es ist zwar eine große Zahl recht milder Tenside erhältlich, jedoch sind die Tenside des Standes der Technik entweder mild, reinigen aber schlecht, oder aber sie reinigen gut, reizen jedoch Haut oder Schleimhäute.

Diesem Übelstande galt es also, Abhilfe zu schaffen.

Die vorliegende Erfindung betrifft in einer besonderen Ausführungsform Reinigungszubereitungen für die Verwendung als Duschpräparat.

Auch derartige Zubereitungen sind an sich bekannt. Es handelt sich dabei im wesentlichen um oberflächenaktive Substanzen oder Stoffgemische, die dem Verbraucher in verschiedenen Zubereitungen angeboten werden. Zubereitungen solcher Art zeichnen sich im Allgemeinen durch einen mehr oder weniger hohen Wassergehalt aus, können aber auch beispielsweise als Konzentrat vorliegen.

Im Allgemeinen unterscheiden sich Präparate, welche für das Duschbad vorgesehen sind, nicht oder kaum von Wannenbadzubereitungen, abgesehen davon, dass bei Duschzubereitungen Produkte höherer Viskosität bevorzugt werden, die nicht nach Entnahme aus dem Behälter aus der Hand rinnen. Dies ist bei Wannenbadzubereitungen weniger von praktischer Bedeutung.

Schon bei einem einfachen Wasserbade ohne Zusatz von Tensiden kommt es zunächst zu einer Quellung der Hornschicht der Haut, wobei der Grad dieser Quellung beispielsweise von der Dauer des Bades und dessen Temperatur abhängt. Zugleich werden wasserlösliche Stoffe, z.B. wasserlösliche Schmutzbestandteile, aber auch hauteigene Stoffe, die für das Wasserbindungsvermögen der Hornschicht verantwortlich sind, ab- bzw. ausgewaschen. Durch hauteigene oberflächenaktive Stoffe werden zudem auch Hautfette in gewissem Ausmaße gelöst und ausgewaschen. Dies bedingt nach anfänglicher Quellung eine nachfolgende deutliche Austrocknung der Haut, die durch waschaktive Zusätze nach verstärkt werden kann.

Bei gesunder Haut sind diese Vorgänge im Allgemeinen belanglos, da die Schutzmechanismen der Haut solche leichten Störungen der oberen Hautschichten ohne weiteres kompensieren können. Aber bereits im Falle nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muss durch externe Maßnahmen regeneriert werden.

Aufgabe der vorliegenden Erfindung war somit, diesem Mangel des Standes der Technik Abhilfe zu schaffen. Weiterhin war eine Aufgabe der Erfindung, Wannen- aber auch Duschbadzubereitungen zur Verfügung zu stellen, welche einesteils hohe Pflegewirkung besitzen, ohne dass andererseits die reinigende Wirkung dahinter zurücksteht.

Die vorliegende Erfindung betrifft ferner waschaktive haarkosmetische Zubereitungen, landläufig als Shampoos bezeichnet. Insbesondere betrifft die vorliegende Erfindung haarkosmetische Wirkstoffkombinationen und Zubereitungen zur Pflege des Haars und der Kopfhaut.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Harnsäure, Xanthin, Keratin, Glycogen, Citronensäure, Milchsäure) durch die Haarwäsche herausgelaugt werden.

Der Stande der Technik ließ es aber an Shampooformulierungen mangeln, welche dem geschädigten Haar in befriedigender Weise Pflege zukommen ließen. Aufgabe war daher, auch diesen Nachteilen des Standes der Technik Abhilfe zu schaffen.

Übliche, und sich gerade in neuerer Zeit immer weiter verbreitende kosmetische und dermatologische Zubereitungsformen sind Gele.

Kosmetische Gele erfreuen sich beim Verbraucher äußerster Beliebtheit. Da sie meistens durchsichtig sind, oftmals eingefärbt aber ebensooft farblos klar sein dürften, bieten sie dem kosmetischen Entwickler zusätzliche Gestaltungsmöglichkeiten, die teilweise funktionalen Charakter haben, teilweise aber auch lediglich der Aufbesserung des äußeren Erscheinungsbildes dienen. So können beispielsweise dem Produkt, welches sich dem Betrachter dann in der Regel in einer durchsichtigen Verpackung darbietet, durch eingearbeitete Farbpigmente, Gasbläschen und dergleichen, oder aber auch größere Objekte, interessante optische Effekte verliehen werden.

Gerade dann, wenn es erwünscht ist, dass das oder die eingearbeiteten Objekte, mögen diese als solche mit dem bloßen Auge als solche erkenntlich sein, mögen sie in mikroskopischen Ausmaßen, aber in interessanter Anordnung - beispielsweise in Form von künstlich erzeugten Farbschlieren - dann doch sichtbare Formen ergeben, so ist es doch wünschenswert, dass diese Objekte in der Gelformulierung ortsfest bleiben und nicht zu Boden sinken oder in irgendeiner Weise in der Formulierungen andere unliebsame Wanderungen vornehmen.

Flüssigkeiten können bezüglich ihrer rheologischen Eigenschaften durch ihr Fließ- und Deformationsverhalten unterschieden werden. Ideal elastische Körper erleiden durch äußere Kräfte eine elastische Deformation, die bei Wegnahme der äußeren Krafteinwirkung ein spontanes, vollständiges Zurückgehen der Deformation bewirkt. Ideal viskose Körper werden durch äußere Kräfte irreversibel in ihrer Form verändert. Die zunehmende Deformation wird als Fließen bezeichnet. Die meisten Flüssigkeiten sind weder ideal viskos noch ideal elastisch, sondern zeigen sowohl viskose als auch elastische Eigenschaften und werden daher als viskoelastische Substanzen bezeichnet.

Im Großteil viskoelastischer Lösungen werden dispergierte Partikel oder Gasbläschen immer sedimentieren bzw. aufsteigen. Sie besitzen eine endliche Strukturrelaxationszeit. Das bedeutet, dass die Netzwerke in diesen Systemen auf eine Deformation mit einer entsprechenden Schubspannung reagieren. Diese wird aber in einer endlichen Zeit auf den Wert Null relaxieren, so dass sich die gesamte Lösung wieder in einem stabilen Ruhezustand ohne Spannung befindet. Dies bedeutet weiter, dass diese Lösungen eine definierte Nullviskosität besitzen und somit bei kleinen Scherraten einen konstanten Viskositätswert erreichen.

Im Gegensatz zu diesen Systemen gibt es aber auch solche, in denen dispergierte Partikel oder Gasbläschen nicht sedimentieren. Es fällt auf, dass diese Systeme erst oberhalb eines charakteristischen Werts fließen. Dieser Wert wird Fließgrenze genannt. Bei näherer Betrachtung der rheologischen Eigenschaften dieser Systeme fällt auf, dass der Speichermodul in ganzen Frequenzbereich unabhängig ist von der Oszillationsfrequenz und immer wesentlich größer ist als der Verlustmodul.

Dagegen erreicht der Betrag der komplexen Viskosität auch bei den kleinsten Frequenzen keinen konstanten Wert, sondern steigt weiter an.

Carbopolgele enthalten Acrylsäurepolymere, welche linear oder quervernetzt aufgebaut sein können und die eine hohe Anzahl von Carboxylgruppen tragen. In gelöster Form binden diese Strukturen Wasser. Die Neutralisation der Carboxylgruppen führt aufgrund deren elektrostatischen Abstoßung zu einer Ausdehnung und damit Quellung der Polymerketten. In diesem Zustand erreichen die Carbopolgele ihre typischen rheologischen Eigenschaften wie z.B. die Erhöhung der Viskosität der kosmetischen Zubereitung und/oder Ausbildung einer Fließgrenze.

Der Effekt der Ausbildung einer Fließgrenze beruht somit auf der elektrostatischen Abstoßung der Carboxylgruppen. Zusätzliche Elektrolyte schirmen diese Ladungen ab. Dadurch kollabieren die Netzwerke, die Fließgrenze bricht zusammen, Partikel oder Gasbläschen können nicht mehr in Schwebe gehalten werden.

Tenside wirken wie Elektrolyte. Daher war es bisher nicht möglich, gut schäumende Reinigungsprodukte mit einem entsprechend hohem Gehalt an Tensid zu formulieren, die klare Carbopol Gele mit Fließgrenze als Basis enthielten.

Der Stand der Technik kennt zwar bereits entsprechende Systeme mit Xanthan Gum (z.B. EP-A 738 509). Diese besitzen aber bezüglich des Hautgefühls während und nach der Anwendung schlechtere kosmetische Eigenschaften. Darüber hinaus können bei gleicher Einsatzkonzentration nur geringere Viskositäten erreicht werden. Die Ausgestaltung eines Gels, welche dazu geeignete Fließeigenschaften aufweist, bietet dem Fachmanne in der Regel keine überaus großen Schwierigkeiten, außer, wenn hohe Tensidkonzentrationen erreicht werden sollen - in der Regel eine Grundanforderung an Reinigungsprodukte. Der Nachteil solch hoher Tensidkonzentrationen ist, dass meistens nur eingetrübte, trübe oder gar opake Produkte erlangt werden.

In der WO 01/ 19946 werden waschaktive Rezepturen offenbart, die neben einem Gelbildner einen Konditionierer enthalten. In der WO 01 /176552 werden waschaktive Rezepturen offenbart, welche eine Kombination bestimmter Verdicker mit Acylglutamaten betrifft. Diese Schriften konnten jedoch nicht den Weg zur vorliegenden Erfindung weisen.

Ein weiterer Nachteil der Zubereitungen des Standes der Technik war die schlechte Kompatibilität der zur Stabilisierung verwendeten Gelbildner mit Elektrolyten im Allgemeinen und ionischen Tensiden im Besonderen. Solche Zubereitungen weisen dementsprechend schlechte Produktleistungen auf, wie beispielsweise schwache Schaumbildung und unangenehmes Hautgefühl. Ferner sind solche Produkte meistens nicht als wirklich klar zu bezeichnen.

Zwar bestehen durchaus elektrolyt- bzw. tensidtolerante Gelbildner, die aber wiederum in der Regel das Hautgefühl stark beeinträchtigen, weil sie in vergleichsweise hohen Konzentrationen eingesetzt werden müssen. Aufgabe der vorliegenden Erfindung war es daher Formulierungen zu finden, die es erlauben elastische, tensidhaltige Gele mit ausreichender Fließgrenze bei gleichzeitiger Vermeidung eines stumpfen Hautgefühls bei und nach der Anwendung herzustellen.

Zudem müssen zur Ausbildung einer Fließgrenze, die ausreicht, um unterschiedliche Partikel, Luftblasen oder Effektstoffe stabil zu suspendieren, Mengen an Gelbildnern eingesetzt werden, die neben der Ausbildung einer Fließgrenze bzw. einer Erhöhung des Elastizitätsmoduls auch zu einem beträchtlichen Anstieg der Produktviskosität führen. Dies beeinträchtigt die Entnahme durch den Verbraucher, die Restentleerung, die Verteilbarkeit des Produktes und das Anschäumen während der Anwendung.

Aufgabe der vorliegenden Erfindung war es daher, Wege zu finden, die es erlauben, elastische, tensidhaltige Gele mit ausreichender Fließgrenze bei gleichzeitig vergleichsweise geringer Viskosität herzustellen.

Auch diesem Nachteil des Standes der Technik galt es also, Abhilfe zu schaffen.

Es hat sich überraschend gezeigt, und darin liegt die Lösung dieser Aufgaben, dass kosmetische und dermatologische waschaktive Zubereitungen, enthaltend
(a) eine wirksame Menge eines oder mehrerer gelbildender Acrylatverdicker,
(b) 4-Hydroxybenzophenon,
(c) Wasser,
(d) gewünschtenfalls weitere Zusatzstoffe, beispielsweise Tenside, Elektrolyte, Konservierungsmittel und/oder andere,
den Nachteilen des Standes der Technik abhelfen.

Es war für den Fachmann daher nicht vorauszusehen gewesen, dass die erfindungsgemäßen Zubereitungen klare Gele mit hervorragenden rheologischen Eigenschaften bilden würden, die sich darüberhinaus auch noch in vorzüglicher Weise als waschaktive Substanzen eignen würden. Den kosmetischen und/oder dermatologischen Reinigungszubereitungen im Sinn der vorliegenden Erfindung liegen einfache und kostengünstige Rezepturen zugrunde. Sie haben gleichzeitig eine gute Schaumentwicklung und eine hohe Reinigungskraft. Die Zubereitungen wirken regenerierend in bezug auf den allgemeinen Hautzustand, vermindern das Trockenheitsgefühl der Haut und machen die Haut geschmeidig.

Ferner sind nach der Lehre der vorliegenden Erfindungen klare Zubereitungen mit hohen Transmissionswerten erhältlich, beispielsweise solchen, die einen Transmissionswert >30% besitzen.

4-Hydroxyacetohenon stellt ein bekanntes und hochwirksames Antioxidans dar, welches unter anderem von der Gesellschaft Symrise unter der Handelsbezeichnung "Symsave® H" verkauft wird. Es hat die CAS-Nr. 99-93-4 und zeichnet sich durch folgende chemische Struktur aus:

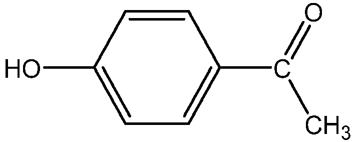

Bei Befolgen der erfindungsgemäßen Lehre sind verdickte Tensidsysteme, insbesondere kosmetische Reinigungsmittel, mit verbesserter Stabilität und Transparenz erhältlich.

Bevorzugte Einsatzkonzentrationen von 4-Hydroxyacetophenon in kosmetischen oder dermatologischen Zubereitungen werden aus dem Bereich von 0,001% bis 2% gewählt, bevorzugt von 0,01% bis 1%, insbesondere bevorzugt von 0,1% bis 0,6%, jeweils bezogen auf das Gesamtgewicht der Zubereitungen.

Als erfindungsgemäß vorteilhaft einzusetzender Acrylatverdicker werden vorteilhaft folgende Substanzen eingesetzt: lineare Polyacrylate, welche als allgemein Carbomere bekannt sind (zum Beispiel Carbopol® Ultrez 10 Polymer, Carbopol® Ultrez 30 Polymer oder Carbopol® 980 Polymer von der Gesellschaft Lubrizol) sowie *Acrylates*/ *C10-30 Alkyl Acrylate Crosspolymere* (zum Beispiel Carbopol® Ultrez 20 Polymer, Carbopol® Ultrez 21 Polymer, Carbopol® ETD 2020 Polymer, Carbopol® 1382 Polymer oder Carbopol® 5984 Polymer von der Gesellschaft Lubrizol).

Weiterhin erfindungsgemäß vorteilhaft einzusetzende Acrylatverdicker sind die Substanzen, die von der Gesellschaft Lubrizol unter der Bezeichnung Carbopol® Aqua SF-1 Polymer (Acrylates Copolymer*)* oder Carbopol® Aqua SF-2 Polymer (Acrylates Crosspolymer-4) verkauft werden. Weitere erfindungsgemäße Vertreter dieser Polyacrylatklasse werden in DE 10 2011 078 087 beschrieben. Substanzen dieser Polymerklasse stellen leicht quervernetzte, durch Alkalien quellbare Acrylat-Copolymere dar, welche folgende Strukturkomponenten enthalten,
- saure Vinylmonomere und/oder ihre Salze (wie zum Beispiel Acrylsäure oder Methacrylsäure),
- nichtionische Vinylmonomere (zum Beispiel C1 bis C5 Alkylester einer Acrylsäure),
- ein oder mehrere quervernetzende Monomere und gegebenenfalls
- Monomere, die eine oder mehrere ungesättigte Endgruppe sowie ggf. einen Polyoxyalkylenteil enthalten.

Die Gesamtmenge an einer oder mehreren erfindungsgemäß verwendeten Acrylatverdickern in den fertigen kosmetischen oder dermatologischen Zubereitungen wird vorteilhaft aus dem Bereich von 0,1 bis 8,0 Gew.-%, bevorzugt 0,3 bis 6 Gew.-%, insbesondere bevorzugt 0,5 bis 4 Gew.-% gewählt, bezogen auf das Gesamtgewicht der Zubereitungen.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wässrigem Medium keine Ionen.

### A. Anionische Tenside

Erfindungsgemäß vorteilhaft zu verwendende anionische Tenside sind Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat,
6. Alaninate.

### Carbonsäuren und Derivate, wie

1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,
Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido-MEA-Sulfosuccinat.
sowie
Schwefelsäureester, wie
1. Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Amphotere Tenside

Erfindungsgemäß vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### C. Nicht-ionische Tenside

Erfindungsgemäß vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid,
6. Sucroseester, -Ether,
7. Polyglycerinester, Diglycerinester, Monoglycerinester,
8. Methylglucosester, Ester von Hydroxysäuren.

Die Gesamtmenge an Tensiden in den fertigen kosmetischen oder dermatologischen Zubereitungen wird vorteilhaft aus dem Bereich von 0,1 bis 18 Gew.-%, bevorzugt 1 - bis15 Gew.-%, insbesondere bevorzugt 2 bis 12 Gew.-% gewählt, bezogen auf das Gesamtgewicht der Zubereitungen.

Als bevorzugte weitere Tenside werden vorteilhaft gewählt Laurylethersulfat, Alkylamidopropylbetain und/ oder Alkylpolyglucoside.

Erfindungsgemäß vorteilhaft ist, den Zubereitungen maximal 0,5%, möglichst wenig, am besten gar keine kationischen Tenside zuzufügen.

Die erfindungsgemäßen waschaktiven Zubereitungen zeichnen sich in der Regel durch einen Wassergehalt von 95 bis 5 Gew.-% aus, bezogen auf das Gesamtgewicht der Zubereitungen und stellen Gele dar.

Erfindungsgemäß vorteilhaft können praktisch alle übliche als in wässrigen Systemen nicht- oder schwerlösliche Festkörper gewählt werden. Bevorzugt im Sinne der vorliegenden Erfindung sind beispielsweise Polymerpartikel oder Silikatpartikel mit Abbrasivwirkung (Scrubs) Partikel mit verkapselten Wirkstoffen oder Ölen u.ä. (Kapselmaterialien: Wachs, Polymere, natürliche Polymere, Gefärbte Partikel ohne Wirkstoffe, Perlglanz- oder Trübungsmittel, Pigmente, Puderrohstoffe wie Talkum, Pflanzenfasern und andere mehr.

Vorteilhaft werden die Zubereitungen so ausgestaltet, dass sie eine Fließgrenze von 0,5 bis 20 Pa aufweisen, bevorzugt 1 bis 6 Pa.

Als Fließgrenze wird die kritische Schubspannung der Fließkurve angesehen. Sie kann erfindungsgemäß wie folgt ermittelt werden:
Gemessen wird die Fließkurve auf einem schubspannungsgesteuerten Rheometer bei 25°C ± 1°C mit 25 mm Platte/Platte Geometrie bei einem Spalt zwischen 0,8 mm und 1,2 mm, wobei strukturschonend befüllt wird. Es wird eine geeignete konstante Schubspannungszeitrampe vorgegeben und vor dem Test eine entsprechende Strukturerholungszeit eingehalten und die kritische Schubspannung im Maximum der Fließkurve angegeben.

Vorteilhaft werden die Zubereitungen so ausgestaltet, dass sie einen tan δ von 0,05 bis 0,6 aufweisen, bevorzugt 0,1 bis 0,5.

Unter tan δ wird erfindungsgemäß der Quotient aus dem Verlustmodul und dem Speichermodul verstanden. Der tan δ wird wie folgt ermittelt:
Gemessen werden Verlust- und Speichermodul durch einen dynamischen Frequenztest auf einem schubspannungsgesteuerten Rheometer bei 40 °C ± 1°C mit 25 mm Platte/Platte Geometrie bei einem Spalt zwischen 0,8 mm und 1,2 mm, wobei strukturschonend befüllt wird. Es wird nach dem Stand der Technik der Frequenztest mit einer entsprechenden Strukturerholungszeit vor dem Test durchgeführt und der tan δ im Frequenzbereich zwischen 0,05 rad/s und 3,0 rad/s angegeben, bevorzugt zwischen 0,08 rad/s und 1,0 rad/s.

Die Fließgrenze kann durch Erhöhung der Gelbildnerkonzentration angehoben werden.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Zubereitungen gemäß der Erfindung sind vorteilhaft auf einen pH-Bereich > 4,2 eingestellt, besonders bevorzugt > 5,0 besonders bevorzugt 5,1 - 7,5.

Ein zusätzlicher Gehalt an Antioxidantien ist im Allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die folgenden Beispiele sollen die Verkörperungen der vorliegenden Erfindungen verdeutlichen. Die Angaben beziehen sich stets auf Gewichts-%, sofern nicht andere Angaben gemacht werden.

### Beispiele:

### Reinigungsgele (die Mengenangaben sind Aktivgehalte):

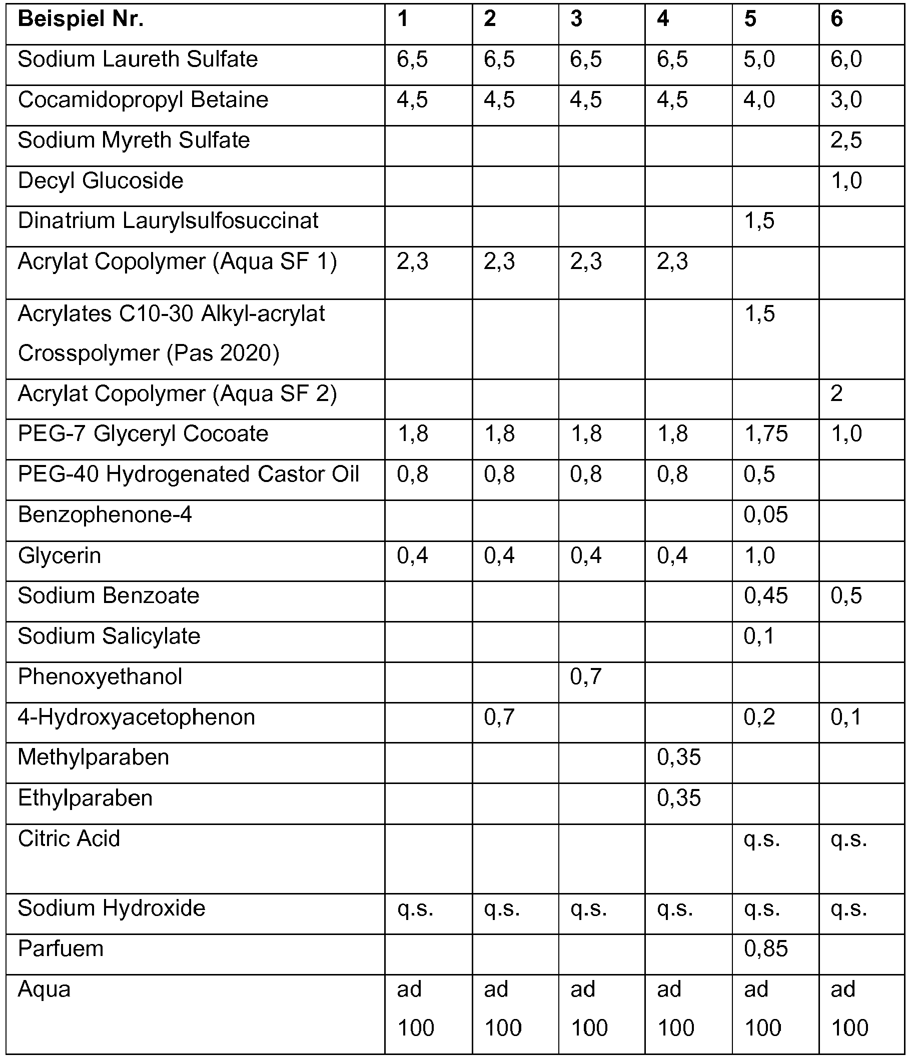

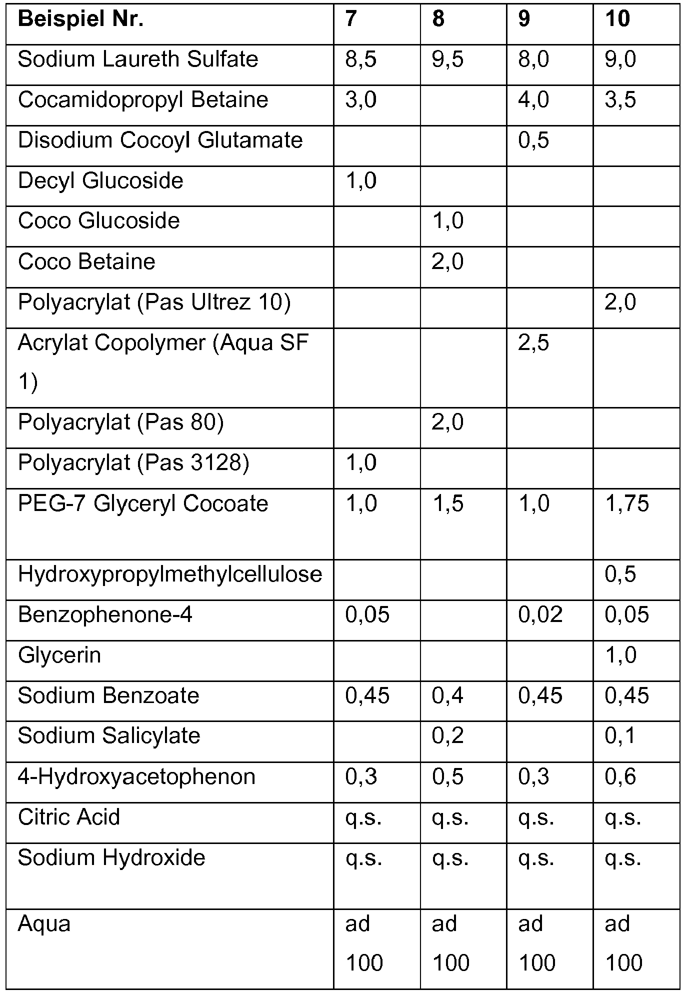

## Patentansprüche

1. Kosmetische und dermatologische waschaktive Zubereitungen in Form klarer Gele, enthaltend
(a) mindestens einen gelbildendenden Acrylatverdicker,
(b) 4-Hydroxyacetophenon,
(c) mindestens ein anionisches Tensid und
(d) Wasser.

2. Zubereitungen nach Anspruch 1, **dadurch gekennzeichnet, dass** sie - bezogen auf das Gesamtgewicht - die gelbildenden Acrylatverdicker in Mengen von 0,1 bis 8 Gew.-% enthalten.

3. Zubereitungen nach den Ansprüchen 1 und/oder 2, **dadurch gekennzeichnet, dass** sie als Acrylatverdicker Polymere enthalten, die ausgewählt sind aus der Gruppe, die gebildet wird von:
1) Acrylates C10-30 Alkylacrylat Crosspolymer;
2) Polyacrylaten;
3) Copolymeren enthaltend
a) ein oder mehrere Acrylatmonomere ausgewählt aus Acrylsäure, Methacrylsäure, Itaconsäure, Fumarsäure, Crotonsäure, Aconitsäure oder Maleinsäure,
b) ein alpha,beta-ethylenisch ungesättigtes Monomer der allgemeinen Formel CH₂=CXY mit X=H, CH₃, -C1-C30-Alkyl, -CH₂-C(=0)0(CH₂-CH₂-0)x-R³, - CH₂-C(=0)NH(CH₂-CH₂-0)x-R³, -CH₂-CH2=(CH2-CH₂-0)x-R³ mit x= 1-100 und R³=C1 - C30 Alkyl oder Cl und Y= -COOR, -C₆H₄R, -CN, -CONH₂, -Cl, - NC₄H₆0, - NH(CH₂)₃COOH, -NHCOCH3, -CONHC(CH₃)₃, -CON(CH₃)₂, - CH=CH₂, C1-C18-Alkyl, Hydroxy-C1 -C18-Alkyl, -C(=0)0(CH₂-CH₂-0)_{X}-R3, -C(=0)NH(CH₂-CH₂-0)ₓ-R³, -CH2=(CH₂-CH₂-0)ₓ-R³ mit x= 1 -100 und R³=C1 -C30-Alkyl oder der Formel CH₂=CH(OCOR²) mit R²=C1-C18 Alkyl oder der Formel CH₂=CH₂ oder CH₂=CHCH₃ und
c) eine mehrfach ungesättigte Komponente, die zur teilweisen Quervernetzung geeignet ist.

4. Zubereitungen nach mindestens einem der Ansprüche 1 bis 3, **dadurch gekennzeichnet, dass** sie - bezogen auf das Gesamtgewicht- 4-Hydroxyacetophenon in Mengen von 0,001 bis 2 Gew.-% enthalten..

5. Zubereitungen nach mindestens einem der Ansprüche 1 bis 4, **dadurch gekennzeichnet, dass** sie die anionischen Tenside - bezogen auf das Gesamtgewicht - in Mengen von 0,1 bis 18 Gew.-% enthalten.

6. Zubereitungen nach mindestens einem der Ansprüche 1 bis 5, **dadurch gekennzeichnet, dass** sie anionische Tenside enthalten, die ausgewählt sind aus der Gruppe, die gebildet wird von:
Dinatrium Acylglutamat,
Dinatrium Laurylsulfosuccinat,
Natrium Laurethsulfat,
Ammonium Laurylsulfat,
Natrium Laurylsulfat,
Natrium Cocosulfat,
Natrium Methylcocoyltaurat,
Natrium Myrethsulfat,
Dinatrium Lauroylglutamat,
Dinatrium Cocoylglutamat,
Dinatrium Stearoylglutamat.

7. Zubereitungen nach mindestens einem der Ansprüche 1 bis 6, **dadurch gekennzeichnet, dass** sie einen Wasseranteil - bezogen auf das Gesamtgewicht - von 5 bis 95 Gew.-% aufweisen.

## Claims

1. Cosmetic and dermatological surface-active compositions in form of clear gels, comprising:
(a) at least one gel-forming acrylate thickener;
(b) 4-hydroxyacetophenone;
(c) at least one anionic surfactants, and
(d) water.

2. Compositions of Claim 1, **characterized in that** they contain said gel-forming acrylate thickeners in amounts of 0.1 to 0.8 percent by weight - calculated on the total amount.

3. Compositions of Claims 1 and/or 2, **characterized in that** they as contain acrylate thickeners polymers selected from the group consisting of:
1) acrylates C10-30 alkylacrylate cross-polymer;
2) polyacrylates;
3) copolymers containing:
(a) one or more acrylate monomers selected from acrylic acid, methacrylic acid, itaconic acid, fumaric acid, crotonic acid, aconitic acid or maleic acid;
(b) an alpha,beta-ethylenically unsaturated monomer of general formula CH₂=CHX with X = H, CH₃, C1-C30 alkyl, -CH₂-C(=0)0(CH₂-CH₂-O)x-R³, -CH₂-C(=0)NH(CH₂-CH₂-0)x-R³, -CH₂-CH₂=(CH₂-CH₂-0)x-R³ with x= 1 to 100 et R³ = C1-C30 alkyl or Cl and Y= -COOR, -C₆H₄R, -CN, -CONH₂, -Cl, -NC₄H₆0, - NH(CH₂)₃COOH, -NHCOCH₃, -CONHC(CH₃)₃, -CON(CH₃)₂, -CH=CH₂, C1-C18 alkyl, C1-C18 hydroxyalkyl, -C(=0)0(CH₂-CH₂-0)x-R³, -C(=0)NH(CH₂-CH₂-0)x-R³, - CH₂=(CH₂-CH₂-0)x-R³ with x= 1 à 100 et R³ = C1-C30 alkyl, or of formula CH₂=CH(OCOR²) with R² = C1-C18 alkyl, or of formula CH₂=CH₂ or CH₂=CHCH₃, and
(c) a polyunsaturated component capable of partial cross-linking.

4. Compositions according to at least one of Claims 1 to 3, **characterized in that** they contain 4-hydroxyacetophenone in amounts of from 0.001 to 2 percent by weight - calculated on the total amount.

5. Compositions according to at least one of Claims 1 to 4, **characterized in that** they contain said anionic surfactants in amounts of 0.1 to 18 percent by weight - calculated on the total amount.

6. Compositions according to t least one of Claims 1 to 5, **characterized in that** they contain anionic surfactants selected from the group consisting of:
disodium acylglutamate,
disodium laurylsulfosuccinate,
sodium laurethsulfate,
ammonium laurylsulfate,
sodium laurylsulfate,
sodium cocosulfate,
sodium methylcocoyltaurate,
sodium myrethsulfae,
disodium lauroylglutamate,
disodium cocoylglutamate,
disodium stearoylglutamate.

7. Compositions according to any of Claims 1 to 6, **characterized in that** they show water content of 5 to 95 percent by weight - calculated on the total amount.

## Revendications

1. Préparations cosmétiques et dermatologiques détergentes sous la forme de gels transparents, contenant :
(a) au moins un épaississant à base d'acrylate formant un gel,
(b) de la 4-hydroxyacétophénone,
(c) au moins un tensioactif anionique et
(d) de l'eau.

2. Préparations selon la revendication 1, **caractérisées en ce qu'**elles contiennent les épaississants à base d'acrylate formant un gel, par rapport au poids total, en quantités de 0,1 à 8 % en poids.

3. Préparations selon les revendications 1 et/ou 2, **caractérisées en ce qu'**elle contiennent en tant qu'épaississants à base d'acrylate des polymères qui sont choisis dans le groupe formé par :
1) Acrylates C10-30 Alkylacrylat Crosspolymer;
2) les polyacrylates ;
3) les copolymères contenant :
a) un ou plusieurs monomères à base d'acrylate choisis parmi l'acide acrylique, l'acide méthacrylique, l'acide itaconique, l'acide fumarique, l'acide crotonique, l'acide aconitique ou l'acide maléique,
b) un monomère alpha,bêta-éthyléniquement insaturé de formule générale CH₂=CXY avec X = H, CH₃, alkyle en C1-C30, -CH₂-C(=0)0(CH₂-CH₂-O)x-R³, - CH₂-C(=0)NH(CH₂-CH₂-0)x-R³, -CH₂-CH₂=(CH₂-CH₂-0)x-R³ avec x= 1 à 100 et R³ = alkyle en C1-C30 ou Cl et Y= -COOR, -C₆H₄R, -CN, -CONH₂, -Cl, -NC₄H₆0, - NH(CH₂)₃COOH, -NHCOCH₃, -CONHC(CH₃)₃, -CON(CH₃)₂, -CH=CH₂, alkyle en C1-C18, hydroxy-alkyle en C1-C18, -C(=0)0(CH₂-CH₂-0)x-R³, -C(=0)NH(CH₂-CH₂-0)x-R³, -CH₂=(CH₂-CH₂-0)x-R³ avec x= 1 à 100 et R³ = alkyle en C1-C30, ou de formule CH₂=CH(OCOR²) avec R² = alkyle en C1-C18, ou de formule CH₂=CH₂ ou CH₂=CHCH₃, et
c) un composant polyinsaturé, qui est approprié pour la réticulation partielle.

4. Préparations selon au moins l'une quelconque des revendications 1 à 3, **caractérisées en ce qu'**elles contiennent la 4-hydroxyacétophénone, par rapport au poids total, en quantités de 0,001 à 2 % en poids.

5. Préparations selon au moins l'une quelconque des revendications 1 à 4, **caractérisées en ce qu'**elles contiennent les tensioactifs anioniques, par rapport au poids total, en quantités de 0,1 à 18 % en poids.

6. Préparations selon au moins l'une quelconque des revendications 1 à 5, **caractérisées en ce qu'**elles contiennent des tensioactifs anioniques qui sont choisis dans le groupe formé par :
l'acylglutamate de disodium,
le laurylsulfosuccinate de disodium,
le laurethsulfate de sodium,
le laurylsulfate d'ammonium,
le laurylsulfate de sodium,
le cocosulfate de sodium,
le méthylcocoyltaurate de sodium,
le myrethsulfate de sodium,
le lauroylglutamate de disodium,
le cocoylglutamate de disodium,
le stéaroylglutamate de disodium.

7. Préparations selon au moins l'une quelconque des revendications 1 à 6, **caractérisées en ce qu'**elles présentent une proportion d'eau, par rapport au poids total, de 5 à 95 % en poids.
